# EUROPEAN PATENT SPECIFICATION

(11) **EP 1 112 747 B1**
(45) Date of publication and mention of the grant of the patent: **16.06.2004**
(21) Application number: 00204630.8
(22) Date of filing: 19.12.2000
(51) Int. Cl.: A61K 39/112, A61P 31/04, C12N 1/20

(54) **Salmonella vaccine not inducing antibodies against flagellin or flagella**
Salmonella Impfstoff, der keinen Antikörper gegen Flagellin oder gegen Flagella induziert
Vaccin contre les Salmonella n'induisant pas d'anticorps réagissant avec la flagelline ou les flagelles

(30) Priority: 28.12.1999 EP 99204564
(43) Date of publication of application: 04.07.2001
(73) Proprietor: Akzo Nobel N.V., 6824 BM Arnhem (NL)
(72) Inventor: Nuijten, Petrus Johannes Maria, 5831 RD Boxmeer (NL); Witvliet, Maarten Hendrik, 5807 BW Oostrum (NL)
(74) Representative: Mestrom, Joannes Jozef Louis

(56) References cited:
- GB-A- 1 109 179
- US-A- 4 886 748
- HE XIAO-SONG ET AL: "Hypervariable region IV of Salmonella gene fliC-d encodes a dominant surface epitope and a stabilizing factor for functional flagella." JOURNAL OF BACTERIOLOGY, vol. 176, no. 8, 1994, pages 2406-2414, XP000946785 ISSN: 0021-9193

## Description

The present invention relates to *Salmonella* bacteria for use in a vaccine, to vaccines based upon these bacteria, to the use of such bacteria for the manufacture of a vaccine and to methods for the preparation of such vaccines.

Bacteria of the genus *Salmonella* are notorious for their pathogenicity in both man and animals. In the USA alone, on a yearly basis the number of humans suffering from *Salmonella* infections exceeds the two million cases. In most cases, the infection is caused by contaminated food. Well-known sources of infection are eggs (from both ducks and chickens), products containing eggs and not sufficiently heated poultry and pig meat. Especially in infants, young children, elderly people and immune compromised patients, the ability to cope with such infections is low. Especially in these groups, the yearly death rate due to *Salmonella* infections is high. During the last few decades the more efficient large-scale animal husbandry has led to an enormous increase in animal density. As a result, an increase is seen of the number of animal infections and subsequent human infections caused by infected food. It is clear that animals are the main source of *Salmonella* infection. This source is very difficult to control. First of all *Salmonella* infections in most cases cause no serious illness in healthy full-grown animals; these animals can carry the bacterium for a prolonged period. During that time they are shedding the bacterium in their dung. This makes it practically impossible to avoid infection in the more vulnerable young animals. Secondly, many *Salmonella* species colonise several different host animal species. Some of the *Salmonella* species cause primary infections in specific hosts, whereas other *Salmonella* species are not restrictive at all. As a primary infectans, *S*. *typhi* and *paratyphi* are frequently associated with infection in man. *S*. *typhi* causes diarrhoea, and as a result, dehydration. This infection is very frequently found in tropical areas. *S*. *dublin* is connected with cattle, specifically young animals, where it causes lethal infections in over 50 % of the cases. *S. abortus-equi* causes abortion in horses. *S. abortus-ovi* causes abortion in sheep. *S. choleraesuis* is the cause of lethal diarrhoea in young pigs. *S. typhimurium* and *S. enteritidis* cause salmonellosis in poultry, pigs, cattle and rodents. *S. arizonae* also causes disease in turkeys. Also non-food animals such as reptiles suffer from *Salmonella* infections, such as *S. arizonae*.

There clearly is a need for efficient *Salmonella* vaccines. Vaccines are needed to protect humans from *Salmonella* infections transmitted from man to man. Also vaccines are needed to protect humans from food-borne *Salmonella* infections. And finally, vaccines are needed to protect animals against *Salmonella* infections.
Currently, several vaccines against various *Salmonella* species are commercially available. These vaccines, although sometimes efficacious from a vaccine point of view, however share a serious disadvantage. They generally induce an antibody population that equals that of an infection with wild type bacteria because they possess the same antigenic load as the wild type bacterium. Therefore, an analysis of the antibodies in the serum of a *Salmonella-*positive animal does not reveal why the animal is positive. This can be due to vaccination, but it can equally well be caused by infection with a virulent field strain. Therefore, if an animal is *Salmonella*-positive, it is considered to be infected. Therefore, it would be desirable to have a so-called marker vaccine. A marker vaccine is a vaccine that can be discriminated from wild type infection, e.g. on the basis of a characteristic antibody panel, different from the antibody panel induced by wild type infection. A different antibody panel is induced e.g. when an immunogenic protein present on a wild type bacterium is not present in a mutant that is used for vaccination: the host will then not make antibodies against that protein after vaccination.
A marker antigen has at least the following characteristics:
⇒ it can be deleted without severely impairing the viability of the bacterium.
⇒ it induces antibodies when present.
⇒ it does, when present, not contribute to the immunogenicity of the bacterium
⇒ its absence preferably contributes to attenuation.

When searching for suitable marker antigens, all known *Salmonella* antigens are to be considered. A known antigen found with all wild type *Salmonella* species with the exception of some *S. pullorum* and *gallinarum* subspecies is the flagellum. Examples of *Salmonella* species carrying flagella when in their wild type form are *S. typhimurium*, *enteritidis, choleraesuis, dublin, typhi, abortus-ovi, abortus-equi, paratyphi A and B, derby, hadar, heidelberg, agona* and *arizonae*. Flagella are long structures protruding from the cell surface, that play an important role in motility and invasion of certain host cells. Flagella consist of long polymers of the protein called flagellin. It is known that these flagella induce high levels of antibodies. It is also known that the absence of flagella does not significantly impair the viability of the bacterium outside the host: flagella-less mutants of practically all *Salmonella* species are known and can be grown *in vitro.* Nevertheless, flagellar proteins of *Salmonella* have never been contemplated as suitable markers, since they do not or only partially fulfill three of the four marker-requirements:
-- although not essential for survival outside the host, they do play a significant role in bacterial survival and persistence in host macrophages, involved in inducing immunity. (Methner, U. and Barrow, P.A., Berl. Münch. Tierärtzl. Wschr. 110: 391-396 (1997), Weinstein et al., Infect. Immun. 46: 819-825 (1984))
-- they strongly contribute to the immunogenicity of the bacterium, a reason why they are even used as carrier proteins for short heterologous amino acid sequences (Joys, T.M., SAAS Bulletin: Biochem. & Biotech. 4: 56-59 (1991), Newton, S.M.C. et al., Science 244: 70-72 (1989))
-- their absence does not contribute to attenuation (Lockman, H.A. and Curtiss, R., Infect. & Immun. 58: 137-143 (1990), Methner, U. and Barrow, P.A., Berl. Münch. Tierärtzl. Wschr. 110: 391-396 (1997)
-- flagellated *Salmonella* bacteria inhibit the binding of other flagellated *Salmonellas.* Flagella-less *Salmonella* bacteria however show a reduced inhibition potential towards other flagellated *Salmonellas,* and therefore even increase the overall level of *Salmonella* infection. (Methner, U. and Barrow, P.A., Berl. Münch. Tierärtzl. Wschr. 110: 391-396 (1997), Weinstein et al., Infect. Immun. 46: 819-825 (1984))
In the English Patent GB 1,109,179, Ephraim Saul Anderson described vaccines on the basis of inactivated *Salmonella typhi* or *paratyphi* that are devoid of flagella. These vaccines were given without adjuvant. As was described by Anderson several years later (Wandan, M. et al., 1975, Bull. World Health Organisation), these vaccines did not provide any protection. Moreover, this paper once more stressed the importance of flagella in vaccines.
For these combined reasons, the flagellum was never considered to be a suitable deletable marker antigen for *Salmonella* vaccines.

Surprisingly it was found now that contrary to the general assumption, the absence of flagella did not affect the vaccinating potential of *Salmonella.* This is especially surprising in view of the fact that in wild type *Salmonella* infected animals large amounts of antibodies against the flagella are found, once more proving their antigenicity.
It is an object of the present invention to provide bacteria of the genus *Salmonella* that in their wild type form carry flagella but are no longer capable to induce antibodies against at least one antigenic determinant of flagellin or flagella, when in an inactivated form together with an adjuvant, for use in a vaccine for the protection of humans and animals against Salmonellosis.

Flagellins are proteins of about 500 amino acids that comprise several antigenic determinants, i.e. there are several regions on the flagellins against which antibodies are raised in the host animal. These antigenic determinants have been identified and localised by T.M. Joys. (Joys, T.M., SAAS Bulletin: Biochem. & Biotech. 4: 56-59 (1991)). For the purpose of the present invention, bacteria that are no longer capable to induce antibodies against at least one antigenic determinant of flagellin or flagella are considered to be bacteria that do not comprise flagellin or flagella that still possesses all the antigenic determinants. It is not necessary to remove all antigenic determinants: it suffices to delete one of the antigenic determinants. Screening for antibodies against this antigenic determinant in the serum of seropositive animals then would allow discrimination between marker-vaccinated and wild type infected animals. Such antibodies would not be found in vaccinated animals, whereas they would be present in naturally infected animals. Screening can easily be done with simple diagnostic tools on a routinely basis as will be described below.
Much is currently known about the synthesis of flagellin and the subsequent maturation into flagella. (E.g. in *Escherichia coli* and *Salmonella typhimurium*. Chapter 10: Flagella and motility. Eds. Frederick C. Neidhardt et al. 2nd. ed. ISBN 1-55581-084-5 (1996) and in Macnab, R.M.; Genetics and biogenesis of bacterial flagella (Annual Review of Genetics 26: 131-158 (1992)). The process of flagellar biogenesis requires the concerted action of a large number of genes, not only the gene encoding the flagellin but also a large number of genes involved in the synthesis of the flagellum and the flagellar motor. In principle, there are two approaches for making bacteria according to the present invention. First of all, mutations can be made in the gene encoding the flagellin protein in order to mutate one or more antigenic determinants. Secondly, one or more genes involved in the biogenesis of the flagellum can be mutated. This will prevent the biosynthesis of flagellin or even the whole flagellum, and in many cases even the transport of the flagellin through the bacterial membrane. In the case that no flagella are produced, antigenic determinants determined by the specific folding of the flagellin in flagella will be absent. If the flagellin itself is not transported through the membrane and thus remains inside the bacterium, there will be no induction of antibodies against flagellin. All kinds of genes or gene clusters known in the art to be involved in assembly and export such as the Morphological Assembly Pathway and a Flagellum-specific Export Pathway can be targets for mutation. They all lead either to bacteria lacking flagellin or at least flagella. For the sake of clarity, all pathways involved in the whole process of synthesis of the final flagella are further referred to as flagellar biogenesis pathways.

Therefore, in a preferred embodiment, the bacterium is not capable to induce antibodies against at least one antigenic determinant of flagellin or flagella due to a mutation in a gene of the flagellar biogenesis pathway.

From a practical point of view, it may however be desirable to delete (part of) the whole flagellin gene from the bacteria to be used in the vaccine, simply by deletion of the gene encoding the flagellar protein. The genes encoding the flagellar proteins of the various *Salmonella* species are known. They are all very closely related, and therefore highly homologous. Flagellin genes have i.a. been described for *Salmonella enterica* (Li, J. et al., Proc. Natl. Acad. Sci. 91, 2552-2556 (1994)), *Salmonella enteritidis* (Selander, R.K. et al., J. Bacteriol. 174, 3587-3592 (1992)), *Salmonella dublin* (Masten, B.J. and Joys, T.M., J. Bacteriol. 175, 5359-5365 (1993)), *Salmonella typhimurium* (de Vries, N. et al., Appl. Environ. Microbiol. 64, 5033-5038 (1998)), *Salmonella abortus-equi* (Hanafusa, T. et al., Mol. Gen. Genet. 236, 260-266 (1993)). Flagellin genes of novel *Salmonella* species can easily be found on the basis of their homology with all existing and known *Salmonella* flagellin genes: standard hybridisation techniques suffice for locating the flagellin gene.

There are two flagellin genes in wild-type flagellum-bearing *Salmonella* bacteria. Only one of these genes is switched on, i.e. produces flagellin at any given time. Due to a mechanism called flagellar phase variation, at a time scale on the order of 10³ to 10⁵ generations the genes change roles so that the non-expressed one becomes expressed and vice versa. (E.g. described in *Escherichia coli* and *Salmonella typhimurium*. Chapter 10: Flagella and motility. Eds. Frederick C. Neidhardt et al. 2nd. ed. ISBN 1-55581-084-5 (1996)). In order to avoid strains according to the invention to start express flagellin after a certain number of generations, both flagellin genes must be made non-functional. Alternatively, if the phase switching mechanism is made non-functional it suffices to knock out the flagellin gene that is expressed at that time.

A non-functional gene is a gene that does no longer encode a flagellin. This can be a gene from which a part of the coding sequence has been removed that encodes an antigenic determinant.

One possible way of making the flagellin gene or any of the other known genes involved in flagellum-biosynthesis non-functional is by means of classical methods such as the treatment of wild-type bacteria flagella-producing bacteria with mutagenic agents such as base analogues, treatment with ultraviolet light or temperature treatment (Anderson, P. 1995. Mutagenesis, p 31-58 in Methods in Cell Biology 48. H.F. Epstein and D.C. Shakes (Eds)). Other methods for making flagella-less mutants of various bacteria of which the wild type has flagella, have been described i.a. by Liu, S. L. et al. (Infect. Immun. 1988 Aug; 56(8): 1967-73), Haas, R. et al., (Mol. Microbiol. 1993 May; 8(4): 753-60) and Graf, J. et al. (J. Bacteriol. 1994 Nov; 176(22): 6986-91).

Selection for flagella-less bacteria is very easily done by light microscopic analysis for the absence or presence of flagella. Selection for bacteria lacking at least one antigenic determinant of flagellin or flagella can be done by means of binding assays with monoclonal antibodies against flagellin or flagella. Such anti-flagellar or anti-flagellin monoclonal antibodies can be made according to standard techniques, i.a. by immunising (with flagella) inbred mice by techniques known in the art (Kohler and Milstein, *Nature*, 256,495-497,1975). Monoclonals thus obtained can be used in binding assays with the mutated bacteria, and those bacteria not binding to a specific monoclonal are the bacteria lacking at least one antigenic determinant of flagellin or flagella.

The nature of the mutation caused by classical mutation techniques is unknown. This may be a point mutation which may, although this is unlikely to happen, eventually revert to wild-type. Therefore transposon mutagenesis is a good alternative. Mutation by transposon mutagenesis, is also a mutagenesis-technique well-known in the art. This is a mutation accomplished at a localised site in the chromosome.
A possibility to introduce a mutation at a predetermined site, rather deliberately than randomly, is offered by recombinant DNA-technology. Such a mutation may be an insertion, a deletion, a replacement of one nucleotide by another one or a combination thereof, with the only proviso that the mutated gene no longer encodes functional flagellin. Such a mutation can e.g. be made by deletion of a number of base pairs. Even very small deletions such as stretches of 10 base pairs can already render flagellin non-functional. Even the deletion of one single base pair may already lead to a non-functional flagellin, since as a result of such a mutation, the other base pairs are no longer in the correct reading frame. Each deletion or insertion of a number of base pairs indivisible by three causes such a frame shift. More preferably, a longer stretch is removed e.g. 100 base pairs. Even more preferably, the whole flagellin gene is deleted. It can easily be seen, that especially mutations introducing a stop-codon in the open reading frame, or mutations causing a frame-shift in the open reading frame are very suitable to obtain a strain which no longer encodes flagellin. Site-directed mutagenesis is the method of choice for making a flagellin gene that lacks one or more specific antigenic determinants. Based upon the map of antigenic determinants made by Joys (Joys, T.M., SAAS Bulletin: Biochem. & Biotech. 4: 56-59 (1991)), mutant flagellin genes can be made from which one or more specific antigenic determinants have been removed. All recombinant DNA techniques for the construction of flagellin-negative mutants are well-known standard techniques. They relate to cloning of the flagellin-gene, modification of the gene sequence by site-directed mutagenesis, restriction enzyme digestion followed by re-ligation or PCR-approaches and to subsequent replacement of the wild type flagellin gene with the mutant gene (allelic exchange or allelic replacement). Standard recombinant DNA techniques such as cloning the flagellin gene in a plasmid, digestion of the gene with a restriction enzyme, followed by endonuclease treatment, re-ligation and homologous recombination in the host strain, are all known in the art and described i.a. in Maniatis/Sambrook (Sambrook, J. *et al*. Molecular cloning: a laboratory manual. ISBN 0-87969-309-6). Site-directed mutations can e.g. be made by means of in vitro site directed mutagenesis using the Transformer® kit sold by Clontech. PCR-techniques are extensively described in (Dieffenbach & Dreksler; PCR primers, a laboratory manual. ISBN 0-87969-447-5 (1995)).

Therefore, in a more preferred form, this embodiment of the invention relates to a bacterium that is not capable to express a flagellin due to a mutation in the flagellin gene.

Most preferably, the bacteria are selected from the group consisting of *S. typhimurium*, *enteritidis, choleraesuis, dublin, typhi, abortus-ovi, abortus-equi, paratyphi A and B, derby, hadar, heidelberg, agona* and *arizonae*.

It is another object of the present invention to provide suitable marker vaccines for the protection of humans and animals against Salmonellosis. Vaccines according to the invention have as a characteristic feature that they comprise bacteria as defined above and a pharmaceutically acceptable carrier. The main advantage of vaccines according to the present invention is, that humans and animals vaccinated therewith can be discriminated from both non-vaccinated humans and animals and wild type *Salmonella* infected humans and animals on the basis of their antibody panel.

Vaccines according to the invention can be in a live attenuated or an inactivated form. In both cases the absence of at least one antigenic determinant of flagella or flagellin will result in an antibody panel after vaccination that can easily be discriminated from that induced after wild-type infection.

Inactivated vaccines have the advantage over life vaccines that they are inherently safe. Therefore, one preferred form of the invention relates to vaccines in which the bacteria are in an inactivated form.

Vaccines according to the invention that are based upon live *Salmonella* bacteria however have an advantage over vaccines comprising inactivated bacteria in that they better mimic the natural infection and therefore trigger the immune system in a better way. They also have an additional important advantage as explained hereafter. The development of live attenuated vaccines in general is difficult and time consuming. Moreover, fine-tuning the degree of attenuation is complex: high virulence causes disease, and low virulence induces insufficient protection. Surprisingly, vaccines according to the invention do not show significant differences in virulence when compared to their flagella-bearing counterparts. In other words, removal of the flagellin gene does not significantly change the level of attenuation. This has the unexpected advantage that both future and approved existing live attenuated *Salmonella* strains suitable for use in vaccines can be used in the present invention as soon as they are made non-capable to induce antibodies against at least one antigenic determinant of flagellin or flagella. Therefore, in another preferred form, the vaccines according to the invention comprise live attenuated bacteria.

Given the large amount of vaccines given nowadays to both pets and farm animals, it is clear that combined administration of several vaccines would be desirable, if only for reasons of decreased vaccination costs. It is therefore very attractive to use live attenuated bacteria as a recombinant carrier for heterologous genes, encoding antigens selected from other pathogenic micro-organisms or viruses. Administration of such a recombinant carrier has the advantage that immunity is induced against two or more diseases at the same time. Live attenuated bacteria for use in a vaccine according to the present invention provide very suitable carriers for heterologous genes. In principle such heterologous genes can be inserted in the bacterial genome at any non-essential site.

Therefore, another embodiment of the invention relates to bacteria according to the invention in which a heterologous gene is inserted. Such a heterologous gene can, as mentioned above, e.g. be a gene encoding an antigen selected from other pathogenic micro-organisms or viruses. Such genes can e.g. be derived from pathogenic herpesviruses (e.g. the genes encoding the structural proteins of herpesviruses), Retroviruses (e.g. the gp160 envelope protein), adenoviruses and the like.
Also a heterologous gene can be obtained from pathogenic bacteria. As an example, genes encoding bacterial toxins such as Actinobacillus pleuropneumoniae toxins, Clostridium toxins, outer membrane proteins and the like are very suitable bacterial heterologous genes.
Another possibility is to insert a gene encoding a protein involved in triggering the immune system, such as an interleukin, Tumor Necrosis Factor or an interferon, or another gene involved in immune-regulation.

The use of the flagellin gene as an insertion site has the advantage that there is no need to find a new insertion site for the heterologous gene and at the same time the flagellin gene is inactivated and the newly introduced heterologous gene can be expressed (in concert with the homologous bacterial genes). The construction of such recombinant carriers can be done routinely, using standard molecular biology techniques such as allelic exchange.

Thus, in a preferred form of this embodiment the heterologous gene is inserted in the flagellin gene. The heterologous gene can be inserted somewhere in the flagellin gene or it can be inserted at the site of the flagellin gene while this gene has been partially or completely deleted.

A vaccine according to the present invention also contains, in addition to the bacterium described above a pharmaceutically acceptable carrier. Such a carrier may be as simple as water, but it may e.g. also comprise culture fluid in which the bacteria were cultured. Another suitable carrier is e.g. a solution of physiological salt concentration.

The useful dosage to be administered will vary depending on the age, weight and animal vaccinated, the mode and route of administration and the type of pathogen against which vaccination is sought. The vaccine may comprise any dose of bacteria, sufficient to evoke an immune response. Doses ranging between 10³ and 10¹⁰ bacteria are e.g. very suitable doses.

If the vaccine according to the invention comprises inactivated bacteria, one or more compounds having adjuvant activity must be added to the vaccine. If the vaccine according to the invention comprises live attenuated bacteria, the use of an adjuvant is optional. Adjuvants are non-specific stimulators of the immune system. They enhance the immune response of the host to the vaccine. Examples of adjuvants known in the art are Freunds Complete and Incomplete adjuvant, vitamin E, non-ionic block polymers, muramyldipeptides, ISCOMs (immune stimulating complexes, cf. for instance European Patent EP 109942), Saponins, mineral oil, vegetable oil, and Carbopol. Adjuvants, specially suitable for mucosal application are e.g. the *E. coli* heat-labile toxin (LT) or *Cholera* toxin (CT). Other suitable adjuvants are for example aluminium hydroxide, aluminium phosphate or aluminium oxide, oil-emulsions (e.g. of Bayol F ^{(R)} or Marcol 52 ^{(R)}, saponins or vitamin-E solubilisate.

Other examples of pharmaceutically acceptable carriers or diluents useful in the present invention include stabilisers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer). Especially when such stabilisers are added to the vaccine, the vaccine is very suitable for freeze-drying or spray-drying.
Therefore, in a more preferred form, the vaccine is in a freeze-dried or spray-dried form.

For administration to animals or humans, the vaccine according to the present invention can be given inter alia intranasally, by spraying, intradermally, subcutaneously, orally, by aerosol or intramuscularly. For application to poultry, wing web and eye-drop administration are additionally suitable.

Another embodiment of the invention relates to the use of bacteria according to the invention for the manufacture of a vaccine for the protection of humans and animals against infection with a *Salmonella* bacterium or the pathogenic effects of infection.

The invention also relates to methods for the preparation of a vaccine according to the invention. Such methods comprise the admixing of bacteria according to the invention and a pharmaceutically acceptable carrier.

A diagnostic test for the screening for the absence/presence of *Salmonella* anti-flagellin or anti-flagella antibodies in sera can be e.g. a simple ELISA-test in which flagella or purified flagellin or a short polypeptide comprising an antigenic fragment thereof is coated to the wall of the wells of an ELISA-plate. Incubation with serum from humans or animals to be tested, followed by e.g. incubation with a labelled antibody against the relevant human or animal antibody can then reveal the presence or absence of antibodies against the flagellin.

Another example of a diagnostic test system is e.g. the incubation of a Western blot comprising flagellin with serum of humans or animals to be tested, followed by analysis of the blot.
The diagnostic tests for the detection of antibodies against *Salmonella* flagellin are preferably in the form of a kit, comprising flagellin in a purified form. The flagellin could e.g. be purified through standard protein separation techniques over a suitable column. Another possibility is separation on a PAGE gel followed by Western-blotting. On the Western-blot, the flagellin will form a specific band, separated from other *Salmonella* protein bands, and thus is also considered to be purified. Also, a pure form of the protein can be obtained by expressing flagellin-encoding nucleic acid sequences.
In principle, the easiest way of making such a diagnostic test system is to use purified whole flagellin as explained above. It is however very well possible to use only part of the flagellin. This with the proviso that the fragment used still comprises an antigenic determinant of the protein. All antigenic determinants of the flagellin will induce antibodies by definition. Therefore, the use of a flagellin fragment comprising even one single antigenic determinant of the flagellin will be capable of binding to anti-flagellin antibodies.
A test that is capable of discriminating between serum from non-infected, infected and vaccinated humans or animals comprises e.g. a well A, coated with flagellin and a well B, coated with another *Salmonella* immunogen, or possibly whole *Salmonella* cells. Serum that reacts with wells A and B indicates a field infection or vaccination with a classical vaccine, whereas serum that reacts only with well B indicates that the animal tested is vaccinated with the marker vaccine.

### EXAMPLE 1

*Salmonella typhimurium* strain STMP, an attenuated strain that has been tested as a live vaccine in poultry and pigs and provides good levels of protection was used as starting material for chemical mutagenesis.

### Chemical mutagenesis

*S. typhimurium* STMP was grown on blood agar medium and checked for a positive O-antigen group B agglutination and H-antigen type 2 agglutination. One colony was inoculated into LB medium and incubated for 20 h at 37°C with aeration. Ten µl overnight culture was diluted in 10 ml LB (3 cultures) and incubated at 37°C for 6 h with aeration until the culture reached an O.D. at 600 nm of 0.5. A sample was taken to determine the number of viable bacteria. To each of the three 10 ml cultures 100 µl trioxalen (chemical mutagens form Sigma; 3 mg/ml in DMSO) was added and the suspension was poured into a 10 cm Ø petri dish. The suspension was irradiated with U.V. (Transilluminator UVP; wavelength 365 nm) for 5, 10, or 15 min at a distance of 20 cm. Ten µl was transferred to 10 ml 0.9% NaCl, 1/10 dilutions were made and 100 µl was plated on blood agar plates in order to determine the survival rate of bacteria in mutagenized cultures. Cultures with survival rates of approximately 3% and 20% were grown in 100 ml LB until an OD at 600 nm of 0.5. Bacteria were collected by centrifugation, resuspended in 5 ml LB and stored in 30% glycerol at -70°C.

### Selection for a non-motile, aflagellated mutant.

The mutagenised bacteria (3% survival; between 5,000 and 10,000 independent mutants) were inoculated from the glycerol stock onto 3 blood agar plates each. Bacteria were collected in LB and the OD at 600 nm was adjusted to 2.17. Six 10-fold dilutions were made in LB and then 50 µl H2-antiserum (Difco) was added to 450 µl bacterial suspension. This selection step with H-antiserum was included in order to enrich the suspension of mutants for aflagellated bacteria. This suspension was incubated at 37°C for 6 h while shaking (250 rpm) and then centrifuged for 1 min at 1000 rpm in an Eppendorf mini centrifuge in order to remove the agglutination-complex. From the supernatant 1, 10, and 100 µl was plated on blood agar plates and incubated 20 h at 37°C.

### Results:

### Identification of a non-motile mutant of S. typhimurium STMP.

Selection for non-flagellated bacteria by incubation with H2 antiserum resulted in growth of approximately 40 colonies on plates (10⁶ dilution) inoculated with the serum-treated mutant suspension (3% survival), whereas no colonies had grown on plates (10⁶ dilution) inoculated with the serum-treated STMP suspension. This result indicated that mutants were present that did not agglutinate with the H*2*-antiserum, possibly being aflagellated.
The 40 colonies were tested for H*2*-agglutination and for motility using light microscopy. All mutants were negative for H2-agglutination, but only one mutant appeared non-motile as observed by light microscopy. This non-motile mutant was positive for group B O-antigen agglutination. The mutant was named STM2000.

### In vitro stability of STM2000, a non-motile mutant of S. typhimurium STMP.

The phenotypic stability of STM2000 was tested by 12 in vitro passages on blood agar plates. In the same experiment, the parent strain, STMP, was also passed 12 times. These cultures were observed by light microscopy and all bacteria in the mutant-culture were still non-motile. STMP bacteria were motile, although not all cells showed the same level of motility. Electron microscopic comparison of STMP and STM2000 confirmed that no flagella were present on mutant bacteria.

### In vitro stability of STM2001, a non-motile mutant of S. typhimurium STMP.

In a different experiment *S. typhimurium* SL3261 (Deposit number SGSC 439, Salmonella Genetic Stock Centre, University of Calgary, Alberta, Canada) was chemically mutagenised with NTG and non-motile mutants were selected as described before. One mutant, STM2001, showed no reaction with a flagellin-specific monoclonal antibody. After 2D protein gel electrophoresis it was shown that STM2001 lacked the flagellin spot of 51 kDa and pI 4.7, as compared to its parent strain.
The genetic stability of this strain was tested by growing for at least 50 generations. After this period, still no revertants were found: all bacteria were still non-motile.
Strain STM2001 has been deposited with the Centraalbureau voor Schimmelcultures (CBS), Oosterstraat 1, PO.box 273, 3740 AG Baarn, The Netherlands, under accession-number CBS 108955.

### EXAMPLE 2

### Experimental design

Vaccines were prepared from a flagellated and a non-flagellated *S. enteritidis* (S.e.) phage type 4 strain. The bacteria were cultured in Tryptose Phosphate Broth, inactivated by the addition of formalin to a final concentration of 0.5%, followed by harvest of the bacterial cells by centrifugation. The cells were resuspended in phosphate buffer saline and formulated into water in oil emulsion vaccines at 5x10⁹ bacteria/ml. Five chickens were injected intramuscularly with the S.e. fla⁺ vaccine and 5 chickens received the S.e. fla⁻ vaccine. The animals were vaccinated with 0.5 ml vaccine at 14 and 18 weeks of age. At 22 weeks of age, the chickens were bled, and serum was tested in a double antibody sandwich blocking ELISA system specific for antibodies to the g,m flagellin of S.e. (Zijderveld, F.G. van *et al.* (1993) Vet. Quart. **15**, 135-137)

### Animals

Commercial laying type chickens, approximately 14 weeks of age were obtained from a *Salmonella* free flock.

### Results:

All 5 chickens that had received the S.e. fla⁺ vaccine seroconverted in the g,m specific ELISA (blocking percentage >60%) whereas the 5 chickens vaccinated with the fla⁻ vaccine remained seronegative.

### EXAMPLE 3

### Experiment 1

### Experimental design

To assess safety, broilers were inoculated orally (1 ml), subcutaneously (0.5 ml) and intramuscularly (0.5 ml) with flagella-positive *Salmonella* typhimurium strain STMP, flagella-negative *Salmonella* typhimurium strain STM2000 or wild-type S. typhimurium (*Salmonella typhimurium*). The animals were observed for one week after inoculation followed by post-mortem examination of the surviving chickens.

### Animals

Commercial broilers, three weeks of age were obtained from a *Salmonella* free flock.

### Results:

Following inoculation, 8 out of 10 animals that had received wild-type *Salmonella typhimurium* died (Table 1). At necropsy, the 2 surviving chickens inoculated with the wild-type strain had swollen livers with necrotic foci, swollen spleens and pericardial edema.

One of the STMP inoculated chickens had a slightly swollen liver and one chicken inoculated with STM2000 had a slightly swollen spleen. No further abnormalities were noted in those two groups.

**Table 1**

| Group | STMP | STM2000 | Wild-type |
|---|---|---|---|
| Dose (CFU/ml) | 10^{8.0} | 10^{7.7} | 10^{8.0} |
| Mortality | 0/10^{a} | 0/10^{a} | 8/10^{b} |
| Groups with different superscripts in a row differ (p<0.5, Fisher exact test) | | | |

### Experiment 2

### Experimental design

To test both safety and efficacy, broilers were inoculated orally at 3 and 15 days of age with either STMP or STM2000 followed by challenge infection with a tetracycline resistant wild-type *Salmonella typhimurium* strain at 22 days of age.
Safety was assessed by clinical observation after vaccination and determination of weight gain. Also, cloacal swabs were taken at days 10 and 22 to determine the presence of the vaccine strains in the intestinal tract. Swabs were used to inoculate Brilliant Green Agars (BGA) directly and after enrichment in Rappaport Vassiliades Broth (RVB).
The animals were observed for one week after challenge infection followed by post-mortem examination of the surviving chickens. The livers, spleens, cloacal swabs and swabs of the caecum contents of the surviving vaccinated chickens were cultured for the challenge strain by direct inoculation on BGA containing tetracycline (BGAtet). In addition, the swabs were incubated in an enrichment medium (buffered peptone water containing tetracycline) followed by plating on BGAtet.

### Animals

Commercial broilers, three days of age were obtained from a *Salmonella* free flock.

### Results:

No clinical abnormalities were observed after the oral vaccinations at 3 and 15 days of age. Also, the average weight gains in the vaccinated groups were not different from the control group (Table 3). Both strains were present in cloacal swabs of the vaccinated animals taken 7 days after vaccination (Table 2). The fact that a larger proportion of the chickens in the STMP inoculated group was culture positive after direct plating indicates that this strain colonises the intestinal tract in higher numbers than the STM2000 strain.

**Table 2**

| | STMP positive swabs | | STM2000 positive swabs | |
|---|---|---|---|---|
| Reisolation | Direct | Enrichment | Direct | Enrichment |
| Day 10 | 15/15^{a} | Not done | 7/15^{b} | 14/15 |
| Day 22 | 10/15^{a} | 15/15^{A} | 4/15^{a} | 14/15^{A} |
| Groups with different superscripts in a row differ (p<0.5, Fisher exact test) | | | | |

One chicken in the STMP group died after challenge infection (7%), compared to no mortality in the STM2000 group and 80% mortality in the unvaccinated controls (Table 3). The surviving chickens in the control group also gained considerable less weight than the vaccinates. The weight gain of the STM2000 vaccinated group after challenge infection was significantly higher than the weight gain of the STMP vaccinated group.

**Table 3**

| Group | STMP | STM2000 | Control |
|---|---|---|---|
| Dose d3 | 10^{8.8} | 10^{8.5} | - |
| Dose d15 | 10^{8.5} | 10^{8.5} | - |
| Weight gain d3-d22 | 636±98^{a} | 594±72^{a} | 624±82^{a} |
| *S. t.* challenge dose d22 | 10^{8.0} | 10^{8.0} | 10^{8.0} |
| Weight gain d22-d29 | 299±45^{a} | 339±42^{b} | 31±39^{c} |
| Mortality | 1/15^{a} | 0/15^{a} | 8/10^{b} |
| Groups with different superscripts in a row differ (p<0.5, Fisher exact test (mortality) or two-sample t test (weight gain) | | | |

As shown in Table 4, there was no difference in the reisolation rates of the challenge organism from the spleen and liver. Colonisation of the intestinal tract, as judged by reisolation from cloacal swabs and caecum contents, was significantly lower in the group vaccinated with STM2000.

**Table 4**

| | *Salmonella typhimurium* (tet¹) positive | | | |
|---|---|---|---|---|
| Group | STMP vaccinated | | STM2000 vaccinated | |
| Reisolation | Direct | Enrichment | Direct | Enrichment |
| Spleen | 2/14^{a} | Not done | 1/14^{a} | Not done |
| Liver | 1/14^{a} | Not done | 0/15^{a} | Not done |
| Cloaca | 7/14^{a} | 14/14^{A} | 3/15^{a} | 7/15^{B} |
| Caecum | 13/14^{a} | 14/14^{A} | 5/15^{b} | 9/15^{B} |
| Groups with different superscripts in a row differ (p<0.5, Fisher exact test) | | | | |

### EXAMPLE 4

### Experimental design

To test efficacy, pigs were inoculated orally with STMP (10^{9.0} CFU) or STM2000 (10^{9.3} CFU) followed by an oral challenge infection with a streptomycin resistant wild-type *Salmonella typhimurium* strain (10^{11.0} CFU) 2 weeks later.
Shedding of the challenge strain was measured by culturing faecal samples on days 5 and 8 post challenge. Approximately 5 grams of faeces were placed in peptone water and incubated for 2 hours. Then, serial ten-fold dilutions were made in RVB medium containing streptomycin, incubated overnight, followed by plating on Tryptose Phosphate Broth containing streptomycin. The maximum dilution containing the challenge strain was used to calculate the reisolation score (reciprocal of the maximum dilution positive).
Eight STMP vaccinated pigs and 8 unvaccinated controls were used in a first experiment. In a second experiment 9 pigs were vaccinated with STM2000 and 9 unvaccinated controls were used.

### Animals

Six weeks old SPF pigs were used in both experiments.

### Results:

As shown in table 5, both vaccine strains were able to reduce faecal shedding of the challenge strain significantly.

**Table 5**

| | Average reisolation score | |
|---|---|---|
| | Day 5 | Day 8 |
| STMP (n=8) | 10^{0.9 a} | 10^{10 a} |
| Control (n=8) | 10^{6.4 b} | 10^{3.8 b} |
| | | |
| STM2000 (n=9) | 10^{1.0 A} | 10^{13 A} |
| Control (n=9) | 10^{50 B} | 10^{4.9 B} |
| Groups with different superscripts differ (p<0.5, Mann-Whithey U test) | | |

### Legend to the figures

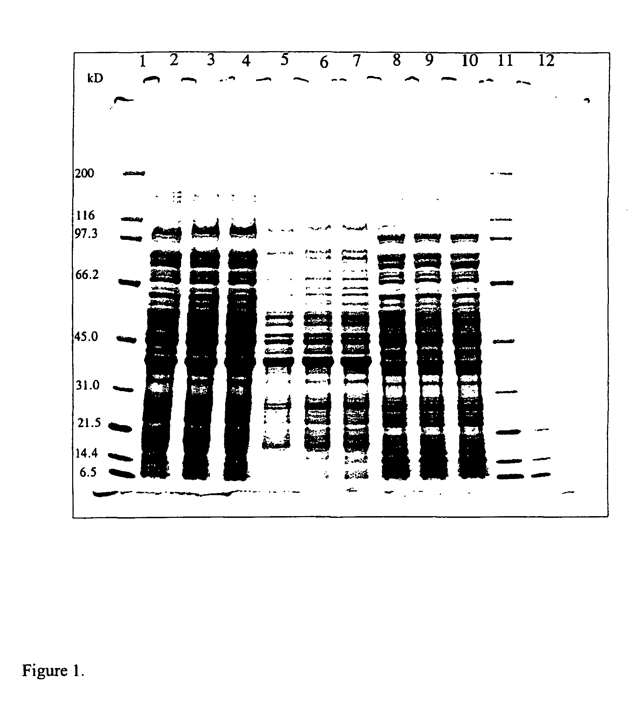

Figure 1: protein profile analysis of STM2000 and its parental strain *S*. *typhimurium* STMP. Lane 1, 11 and 12 molecular weight markers as indicated; lane 2, 5 and 8, *S*. *typhimurium* STMP 12x passed on blood agar medium; lanes 3, 6, and 9, STM2000 12x passed on blood agar medium; lanes 4, 7, and 10, STM2000 2x passed on blood agar medium. Lanes 2, 3, and 4, total protein profile; lanes 5, 6, and 7, pellet after sonication and centrifugation; lanes 8, 9 and 10, supernatant after sonication and centrifugation. Proteins were stained with Coomassie Brilliant Blue.

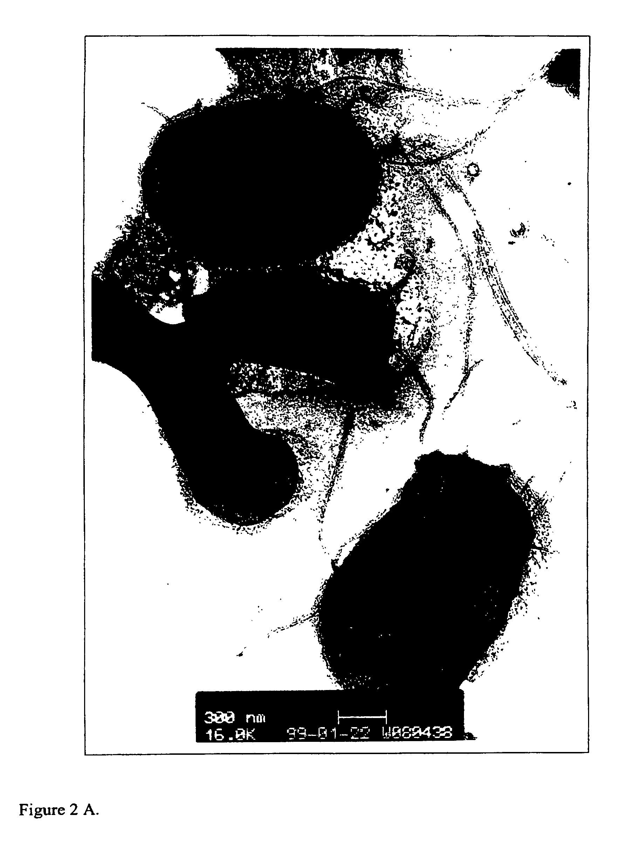

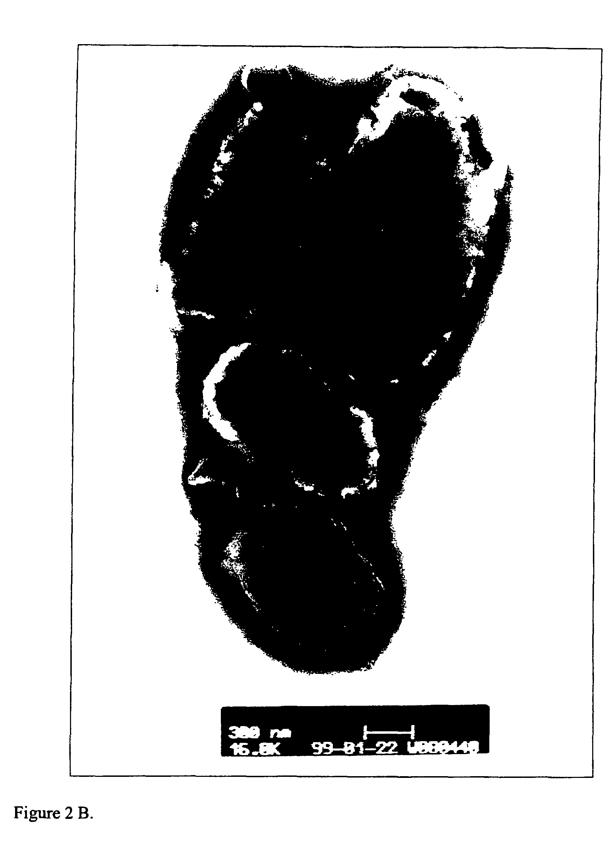

Figure 2: photographs of electron microscopical examination of *S. typhimurium* STMP (A) and its non-motile mutant STM2000 (B).

## Claims

1. Use of a bacterium of the genus *Salmonella* that in its wild type form carries flagella, said bacterium not being capable to induce antibodies against at least one antigenic determinant of flagellin or flagella, and an adjuvant, for the manufacture of an inactivated vaccine for the protection of humans or animals against infection with a *Salmonella* bacterium or the pathogenic effects of infection.

2. Use of a bacterium of the genus *Salmonella* that in its wild type form carries flagella, said bacterium not being capable to induce antibodies against at least one antigenic determinant of flagellin or flagella, for the manufacture of a live attenuated vaccine for the protection of humans or animals against infection with a *Salmonella* bacterium or the pathogenic effects of infection.

3. Use according to claim 1 or 2, wherein said bacterium is not capable to induce antibodies against at least one antigenic determinant of flagellin or flagella due to a mutation in a gene of the flagellar biogenesis pathway.

4. Use according to claim 3, wherein said mutation is located in the flagellin gene.

5. Use according to claims 1-4, wherein said bacterium is selected from the group consisting of *S. typhimurium, enteritidis, choleraesuis, dublin, typhi, abortus-ovi, abortus-equi, paratyphi A and B, derby, hadar, heidelberg, agona* and *arizonae*.

6. Use according to claim 1-5, wherein said bacterium further carries a heterologous gene, said heterologous gene preferably being inserted in the flagellin gene.

7. Vaccine for the protection of animals against Salmonellosis, **characterised in that** the vaccine comprises inactivated bacteria as described in claims 1 or 3-5, an adjuvant and a pharmaceutically acceptable carrier.

8. Vaccine for the protection of animals against Salmonellosis, **characterised in that** the vaccine comprises live attenuated bacteria as described in claims 2-5 and a pharmaceutically acceptable carrier.

9. Vaccine according to claim 7 or 8, **characterised in that** it is in a freeze-dried or spray-dried form.

## Patentansprüche

1. Verwendung eines Bakteriums des Genus *Salmonella,* das in seiner Wildtyp-Form Flagella trägt, wobei das besagte Bakterium nicht fähig ist, Antikörper gegen mindestens eine antigene Determinante von Flagellin oder Flagella zu induzieren, und eines Adjuvans in der Herstellung eines inaktivierten Impfstoffes für den Schutz von Menschen oder Tieren gegen eine Infektion mit einem *Salmonella* Bakterium oder den pathogenen Auswirkungen der Infektion.

2. Verwendung eines Bakteriums des Genus *Salmonella,* das in seiner Wildtyp-Form Flagella trägt, wobei das besagte Bakterium nicht fähig ist, Antikörper gegen mindestens eine antigene Determinante von Flagellin oder Flagella zu induzieren, in der Herstellung eines lebenden attenuierten Impfstoffes für den Schutz von Menschen oder Tieren gegen eine Infektion mit einem *Salmonella* Bakterium oder den pathogenen Auswirkungen der Infektion.

3. Verwendung gemäss Anspruch 1 oder 2, worin das besagte Bakterium aufgrund einer Mutation in einem Gen des flagellaren Biosyntheseweges nicht fähig ist, Antikörper gegen mindestens eine antigene Determinante von Flagellin oder Flagella zu induzieren.

4. Verwendung gemäss Anspruch 3, worin die besagte Mutation im Flagellin-Gen lokalisiert ist.

5. Verwendung gemäss Ansprüchen 1-4, worin das besagte Bakterium ausgewählt ist aus der Gruppe bestehend aus *S*. *typhimurium, enteritidis, choleraesuis, dublin, typhi,* *abortus-ovi, abortus-equi, paratyphi A und B*, *derby, hadar, heidelberg, agona* und *arizonae*.

6. Verwendung gemäss Ansprüchen 1-5, worin das besagte Bakterium ferner ein heterologes Gen trägt, wobei das besagte heterologe Gen vorzugsweise im Flagellin-Gen eingefügt ist.

7. Impfstoff für den Schutz von Tieren gegen Salmonellosis, **dadurch gekennzeichnet, dass** der Impfstoff inaktivierte Bakterien gemäss Ansprüchen 1 oder 3-5, ein Adjuvans und einen pharmazeutisch verträglichen Träger umfasst.

8. Impfstoff für den Schutz von Tieren gegen Salmonellosis, **dadurch gekennzeichnet, dass** der Impfstoff lebende attenuierte Bakterien gemäss Ansprüchen 2-5 und einen pharmazeutisch verträglichen Träger umfasst.

9. Impfstoff gemäss Anspruch 7 oder 8, **dadurch gekennzeichnet, dass** er in einer gefriergetrockneten oder sprühgetrockneten Form vorliegt.

## Revendications

1. Utilisation d'une bactérie du genre *Salmonella* qui, sous sa forme de type sauvage, porte des flagelles, ladite bactérie n'étant pas capable d'induire des anticorps contre au moins un déterminant antigénique de flagelline ou de flagelles, et un adjuvant, pour la fabrication d'un vaccin inactivé destiné à protéger les humains ou les animaux contre l'infection par une bactérie *Salmonella* ou contre les effets pathogènes de l'infection.

2. Utilisation d'une bactérie du genre *Salmonella* qui, sous sa forme de type sauvage, porte des flagelles, ladite bactérie n'étant pas capable d'induire des anticorps contre au moins un déterminant antigénique de flagelline ou de flagelles, pour la fabrication d'un vaccin vivant atténué destiné à protéger les humains ou les animaux contre l'infection par une bactérie *Salmonella* ou contre les effets pathogènes de l'infection.

3. Utilisation selon la revendication 1 ou 2, dans laquelle ladite bactérie n'est pas capable d'induire des anticorps contre au moins un déterminant antigénique de flagelline ou de flagelles du fait d'une mutation dans un gène de la voie de biogenèse flagellaire.

4. Utilisation selon la revendication 3, dans laquelle ladite mutation est située dans le gène de la flagelline.

5. Utilisation selon les revendications 1 à 4, dans laquelle ladite bactérie est choisie parmi le groupe constitué de *S. typhimurium, enteritidis, choleraesuis*, *dublin, typhi, abortus-ovi, abortus-equi, paratyphi A* et *B, derby, hadar, heidelberg, agona* et *arizonae*.

6. Utilisation selon les revendications 1 à 5, dans laquelle ladite bactérie porte en outre un gène hétérologue, ledit gène hétérologue étant de préférence inséré dans le gène de la flagelline.

7. Vaccin pour la protection des animaux contre la salmonellose, **caractérisé en ce que** le vaccin comprend des bactéries inactivées comme décrit dans la revendication 1 ou 3 à 5, un adjuvant et un support pharmaceutiquement acceptable.

8. Vaccin pour la protection des animaux contre la salmonellose, **caractérisé en ce que** le vaccin comprend des bactéries vivantes atténuées comme décrit dans les revendications 2 à 5 et un support pharmaceutiquement acceptable.

9. Vaccin selon la revendication 7 ou 8, **caractérisé en ce qu'**il se présente sous une forme lyophilisée ou séchée par pulvérisation.
